# EUROPEAN PATENT SPECIFICATION

(11) **EP 4 090 786 B1**
(45) Date of publication and mention of the grant of the patent: **18.06.2025**
(21) Application number: 21702854.7
(22) Date of filing: 12.01.2021
(51) Int. Cl.: C23C 14/06, A61L 27/30, C23C 14/14, A61L 29/16, A61L 27/54, C04B 35/58

(54) **HIGH COHESIVE STRENGTH HARD COATINGS CONTAINING SOFT METAL**
HOCHKOHÄSIVE HARTBESCHICHTUNGEN MIT WEICHMETALL
REVÊTEMENTS DURS À FORCE COHÉSIVE ÉLEVÉE CONTENANT UN MÉTAL MOU

(30) Priority: 15.01.2020 US 202062961291 P
(43) Date of publication of application: 23.11.2022
(73) Proprietor: Oerlikon Surface Solutions AG, Pfäffikon, 8808 Pfäffikon, SZ (CH)
(72) Inventor: DRABIK, Martin, 7320 Sargans (CH); MANNINEN, Noora, 7000 Chur (CH); ACIKGOZ DOROKIN, Canet, 8050 Zürich (CH)
(74) Representative: Misselhorn, Hein-Martin
(86) International application number: PCT/EP2021/000002
(87) International publication number: WO 2021/144142

(56) References cited:
- EP-A1- 3 566 725
- US-A1- 2013 224 274
- KELLY P J ET AL: "A study of the antimicrobial and tribological properties of TiN/Ag nanocomposite coatings", SURFACE AND COATINGS TECHNOLOGY, ELSEVIER, NL, vol. 204, no. 6-7, 25 December 2009 (2009-12-25), pages 1137 - 1140, XP026770405, ISSN: 0257-8972, [retrieved on 20090514], DOI: 10.1016/J.SURFCOAT.2009.05.012

## Description

### Field of the Invention

The present invention relates to a hard coating, (e.g. metal nitrides, carbides, oxides or combination of those (e.g. metal carbonitrides) being the metal Ti, Zr, Ta, Al, Si, Cr, or a combination of those metals (e.g. TiN or TiZrN)), containing a soft metal (the soft metal is Ag).

The present invention relates furthermore to a method for producing such soft metal containing hard coatings layers.

### Background of the invention

It is well known that soft metal (e.g. Ag, Au, Cu, Zn) containing hard coatings produced by physical vapor deposition (PVD) techniques can exhibit antibacterial activity; furthermore, the addition of soft metals to hard coatings can enhance the tribological properties due to the reduction of friction coefficient promoted by the presence of soft metals like Ag, Au, Cu and Zn. It is also known that incorporation of soft metals can increase the electrical conductivity of hard coatings, while keeping the wear and mechanical properties characteristic from hard coatings. Hard coatings containing Ag are the most well-known and widely described in literature.

It is also known to have an amount of Ag to be incorporated in the coating within a range of about 2 at.% to 15 at.%, as described by Colmenares Mora et al in U.S. Patent No. 10,143,196B2, in order to obtain acceptable coating adhesion, coating hardness and coating roughness values. In addition the incorporation of Ag above 15 at.% results in a change in coating color, which in some applications (e.g. surgical instruments) is not a desired effect. Publication Kelly P J et al.: "A Study of the antimicrobial and tribological properties of TiN/Ag nanocomposite coatings", Surface and Coatings Technology, Elsevier, NL, Vol:204, Nr:6-7, 25December 2009 (2009-12-25), pages:1137 - 1140, XP026770405, ISSN 0257-8972, DOI: 10.1016/j.surfcoat.2009.05.012, [retrieved on 2009-05-14] relates to antimicrobial and tribological properties of TiN/Ag nanocomposite coatings.
US 2013/224274 A1 relates to a medical product, comprising an antibacterial hard material coating, which is applied to a main body and which comprises biocide. Said hard material coating includes at least one inner layer and one outer layer, wherein the biocide concentration in the outer layer is substantially constant and greater than the biocide concentration in the inner layer and the biocide concentration in the inner layer is greater than or equal to 0.2 at %. EP 3 566 725 A1 describes a coating for an implant component, in particular a component of a spinal implant. The coating is a ceramic titanium nitride coating which, in addition to an at% Ti and an at% N content, has an at% Ag content of 5-30 at%.

### Summary of the invention

An objective of the present invention is to provide a method involving PVD techniques for producing soft metal (Ag) containing hard coatings with enhanced cohesive strength, which is of major importance for applications where high load-bearing capacity or protection against wear is required. The invention is defined by the subject-matter of independent claim 1. The dependent claims are directed to advantageous embodiments.

The present invention provides method for introducing a soft metal into a hard coating during a physical vapor deposition process. The method including steps of providing a substrate; depositing a bonding layer on the substrate; and depositing the hard coating on the bonding layer using vapor deposition wherein the soft metal forms islands in the hard coating.

In the foregoing method, the hard coating is TiN and the metal is Ag.

In the foregoing method, the coating comprises an AgTiN coating and a top layer comprising TiN.

In the foregoing method, the Ag is deposited in islands in the AgTiN coating.

### Brief Description of the Drawings

Preferred embodiments are disclosed and described in detail herein with reference to the accompanying drawings which form a part hereof, and wherein:
FIG. 1 is a schematic representation of a coating according to the present invention;
FIG. 2A is a SEM cross-sectional micrograph of a layer like AgTiN (LL-AgTiN) in total cross-section;
FIG. 2B is a SEM cross-sectional micrograph of a layer like AgTiN (LL-AgTiN) providing a detail view of LL-AgTiN layer of FIG. 2a;
FIG. 3A is a STEM cross-sectional micrograph of a layer like AgTiN (LL-Ag-TiN) showing an overall view of LL-AgTiN and top TiN layer;
FIG. 3B is a STEM cross-sectional micrograph of a layer like AgTiN (LL-Ag-TiN) showing a detailed view of LL-AgTiN layer with identification of chemical compounds from EDS analysis;
FIG. 4A is a SEM cross-sectional micrograph of an island like AgTiN (IL-AgTiN) illustrated in total cross-section;
FIG. 4B is a SEM cross-sectional micrograph of an island like AgTiN (IL-AgTiN) illustrating a detail view of the IL-AgTiN layer of FIG. 4A;
FIG. 5A is a STEM cross-sectional micrograph of a layer like AgTiN (IL-Ag-TiN) showing an overall view of IL-AgTiN and top TiN layer;
FIG. 5B is a STEM cross-sectional micrograph of a layer like AgTiN (IL-Ag-TiN) showing a detailed view of IL-AgTiN layer with identification of chemical compounds from EDS analysis;
FIG. 6A is a Rockwell test (HRC Rockwell value) performed on an alloyed cold-work steel. 1.2842 on LL-AgTiN;
FIG. 6B is a Rockwell test (HRC Rockwell value) performed on an alloyed cold-work steel. 1.2842 on IL-AgTiN;
FIG. 7A is a light optical micrograph of a scratch test indents on layer-like AgTiN (LL-AgTiN) coating on alloyed cold-work steel 1.2842 illustrating a first spallation at 39 N;
FIG. 7B is a light optical micrograph of a scratch test indents on layer-like AgTiN (LL-AgTiN) coating on alloyed cold-work steel 1.2842 illustrating a scratch end at 60 N;
FIG. 8A is a light optical micrograph of a scratch test indents on layer-like AgTiN (IL-AgTiN) coating on alloyed cold-work steel 1.2842 illustrating a first spallation at 35 N;
FIG. 8B is a light optical micrograph of a scratch test indents on layer-like AgTiN (IL-AgTiN) coating on alloyed cold-work steel 1.2842 illustrating a scratch end at 60 N;

### Description of Preferred Embodiment

Layer-like AgTiN "LL-AgTiN" are not according to the invention while island-like AgTiN "IL-AgTiN" are according to the invention.

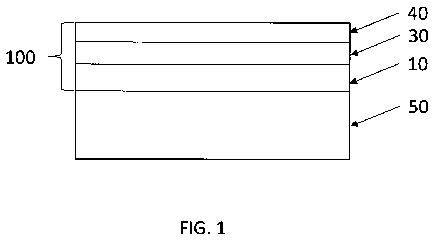

Referring now to the drawings, FIG. 1 shows a schematic representation of a coating according to the present invention wherein a coating 100 is applied to a substrate 50. It is contemplated that besides the amount of Ag incorporated in the coating 100, also the distribution of Ag within the coating 100 plays a major role in coatings properties.

The present invention, in general, provides a coating method for producing soft metal (Ag) containing hard coatings with enhanced cohesive strength, which is obtained by tailoring the soft metal distribution within the hard coating.

In the embodiment described herein, the coating 100 is a AgTiN coating, comprising three major layers: a bonding layer 10 coated between the substrate 50 and an AgTiN coating 30, the AgTiN layer 30 and a top layer comprising TiN 40, as illustrated in Figure 1.

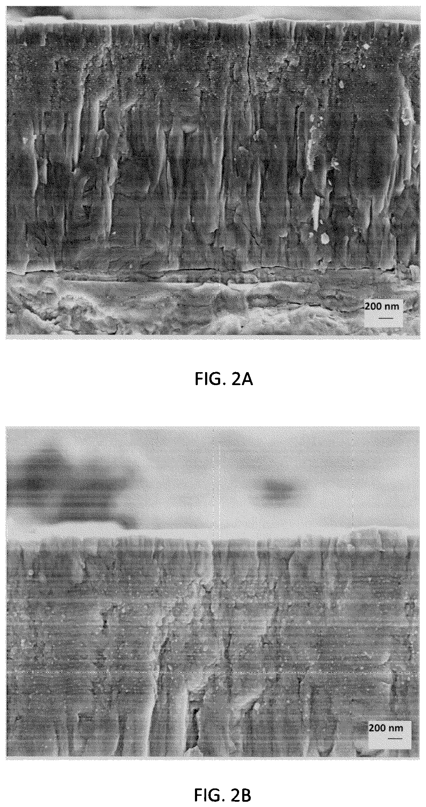

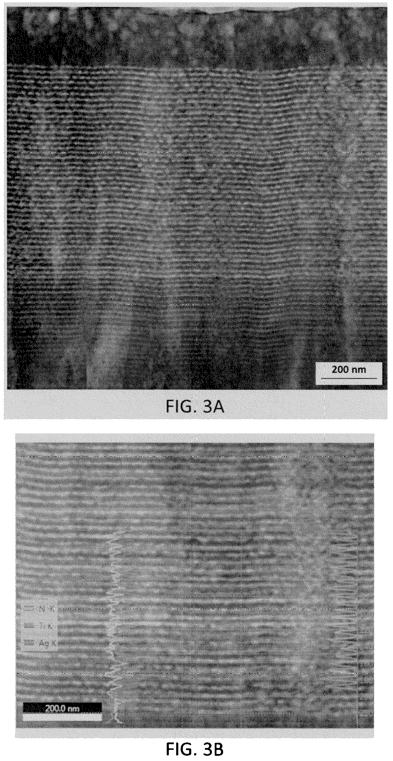

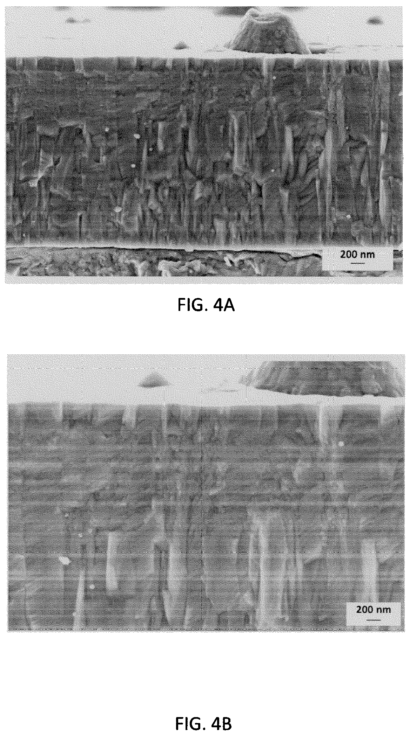

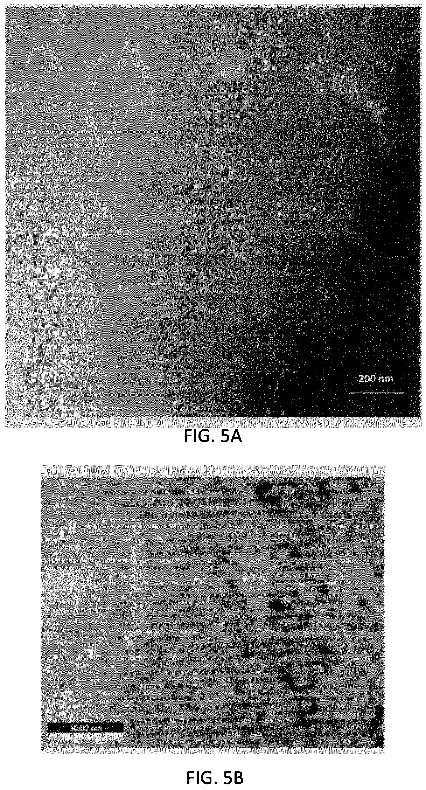

The growth of Ag structures within a hard coating can be tailored: (i) in order to allow formation of continuous Ag layers (see Figures 2A and 2B and Figures 3A and 3B) or (ii) in order to allow the formation of Ag islands (see Figure 4A and 4B and Figures 5A and 5B). The coating on Figures 2A and 2B and Figures 3A and 3B is termed as layer-like AgTiN ("LL-AgTiN") due to the formation of continuous Ag layers alternating with continuous TiN layers. An estimated thickness of each individual Ag layer is on the order of 10 nm, as determined in scanning transmission electron microscopy/electron dispersive spectroscopy profiles (STEM/EDS) analyses. The presence of Ag layers can be already detected in scanning electron microscopy (SEM) cross-sectional analysis. In Figures 2A and 2B, the Ag is shown as the brighter layers/spots. The formation of Ag layers is more clearly observed in STEM analyses (Figures 3A and 3B), where Ag layers can be seen as brighter layers in relation to TiN, due to higher atomic mass of Ag. The presence of Ag layers can also be easily identified in Figure 3B with support of EDS profiles.

The coating in Figures 4A and 4B and Figures 5A and 5B is termed as island like AgTiN ("IL-AgTiN") due to the formation of Ag islands, instead of continuous Ag layers. Contrary to LL-AgTiN, no signs of Ag are visible in SEM analysis. The AgTiN layer shows a more compact structure and morphology due to the impact of Ag on TiN growth, however no Ag layers or large particles are visible in SEM analysis. The presence of Ag is detected only by STEM analysis in Figures 5A and 5B. In Figure 5A the presence of Ag in layered structure is visible, however much less pronounced in comparison to LL-AgTiN shown in Figure 3A. The Ag islands become more visible in Figure 5B. For IL-AgTiN the Ag only becomes visible at very high magnifications, indicating that the Ag does not form continuous layers but islands. The main difference between the LL-AgTiN and IL-AgTiN is that the Ag inclusions form continuous layers in case of LL-AgTiN, while in IL-AgTiN the layer formation is interrupted in order to avoid the full coalescence of Ag and consequent formation of continuous Ag layers.

If, as described above the Ag layer formation is interrupted and islands are formed the main consequence is detected in coating cohesive strength, which is highly improved on island-like IL-AgTiN coating version as compared to layer-like AgTiN (LL-AgTiN).

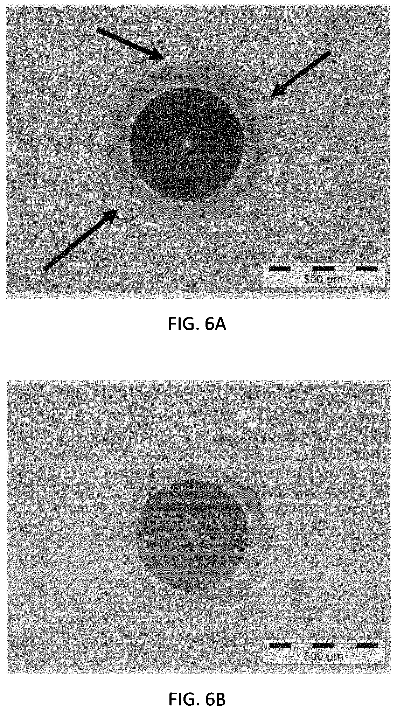

Figures 6A and 6B show light optical micrographs (LOM) of LL-AgTiN coating (Figure 6A) and IL-AgTiN coating (Figure 6B) after HRC Rockwell test, performed on alloyed cold-work steel 1.2842, following guidelines 3198 and 3824-4 of the VDI (Association of German Engineers).

The Rockwell indentation tests shown in Figures 6A and 6B show that no adhesive failure (detachment from substrate) is observed in any of the coatings. The coating with layer-like growth (LL-AgTiN) shown in Figure 6A shows large areas with cohesive flaking in the edge area of the indentation, as indicated by the arrows. Conversely, no cohesive flaking is observed in island-like AgTiN (IL-AgTiN) coating shown in Figure 6B.

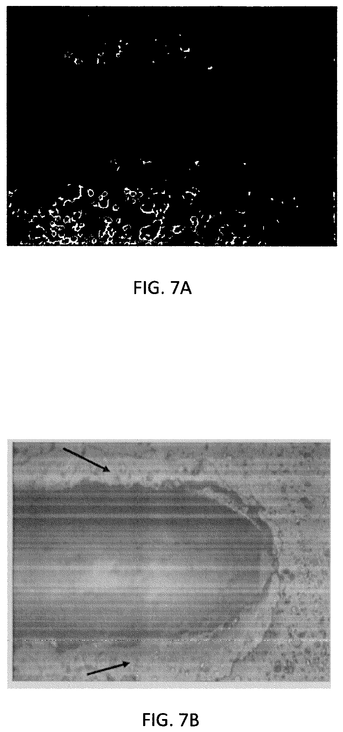

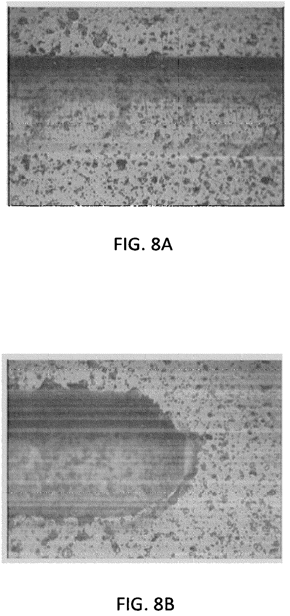

Figures 7A and 7B and Figures 8A and 8B show light optical micrographs (LOM) of LL-AgTiN coating (Figures 7A and 7B) and IL-AgTiN coating (Figures 8A and 8B) after scratch testing, performed on alloyed cold-work steel 1.2842, following guidelines ASTM C1624-05. Scratch testing was performed with an incremental load, starting at 5 N and ending at 60 N, applied at 100 N/min with a speed of 10 mm/min. A diamond indenter with radius of 200 µm was used. Six measurements were performed per coating in order to get statistical analysis.

Both coatings LL-AgTiN (Figures 7A and 7B) and IL-AgTiN (Figures 8A and 8B) show similar values of critical loads with Lc2 at 38.9±2.8 N for LL-AgTiN and 35.3±3.1 N for IL-AgTiN, being the Lc3 >60N for both coatings. Despite no visible delamination of the coating from the substrate, LL-AgTiN shows high areas of cohesive flaking at the end of the scratch, which is not visible on IL-AgTiN, again indicating a higher cohesive strength on island-like AgTiN coating in comparison to layer-like AgTiN.

The deposition of hard coatings (e.g. TiN) containing soft metal (e.g. Ag) can result in different distribution of soft metal in hard coating. The distribution of soft metal within the hard coating plays a major role on coating cohesive strength. The growth of soft metal in island-like structure allow to improve the cohesive strength of the coating. Conversely the formation of continuous soft metal layers alternating with hard layers can result in poor cohesive strength, especially in cases where the soft metal does not form strong bonds with the hard coating.

A hard coating containing an island-like soft metal inclusions can be particularly suitable for applications where antibacterial activity (provided by the soft metal) and resistance to wear under high loads is required (e.g. articulating medical implants). The increase of electrical conductivity is also an advantage in some applications (e.g. sensors) where the wear and mechanical properties of hard coatings are required.

Illustrative embodiments have been described, hereinabove. It will be apparent to those skilled in the art that the above apparatuses and methods may incorporate changes and modifications without departing from the scope of this disclosure. The invention is therefore not limited to particular details of the disclosed embodiments, but is limited to the scope of the claims.

## Claims

1. A method for introducing a soft metal into a hard coating during a physical vapor deposition process, said method comprising steps of:
providing a substrate;
depositing a bonding layer on the substrate; and
depositing the hard coating on the bonding layer using vapor deposition wherein the soft metal being Ag is deposited to start layer formation and the layer formation is interrupted in order to avoid full coalescence in such way that the soft metal forms islands contained in the hard coating being metal nitrides, carbides, or combination of those with metals Ti, Zr, Ta, Al, Si, Cr.

2. The method of claim 1, wherein the hard coating is TiN.

3. The method of claim 2, wherein the coating comprises an AgTiN coating and a top layer comprising TiN.

4. The method of claim 3, wherein the Ag is deposited in islands in the AgTiN coating.

## Patentansprüche

1. Ein Verfahren zum Einbringen eines Weichmetalls in eine Hartbeschichtung während eines physikalischen Gasphasenabscheidungsprozesses, wobei das Verfahren folgende Schritte umfasst:
Bereitstellen eines Substrats;
Abscheiden einer Haftschicht auf dem Substrat; und
Abscheiden der Hartbeschichtung auf der Haftschicht mittels Gasphasenabscheidung,
wobei das Weichmetall Silber (Ag) zur Einleitung der Schichtbildung abgeschieden wird und die Schichtbildung unterbrochen wird, um eine vollständige Koaleszenz zu vermeiden, sodass das Weichmetall Inseln bildet, die in der Hartbeschichtung enthalten sind, wobei die Hartbeschichtung aus Metallnitriden, -carbiden oder einer Kombination daraus mit den Metallen Ti, Zr, Ta, Al, Si, Cr besteht.

2. Verfahren nach Anspruch 1, wobei die Hartbeschichtung TiN ist.

3. Verfahren nach Anspruch 2, wobei die Beschichtung eine AgTiN-Beschichtung und eine Deckschicht aus TiN umfasst.

4. Verfahren nach Anspruch 3, wobei das Silber in Form von Inseln in der AgTiN-Beschichtung abgeschieden ist.

## Revendications

1. Procédé d'introduction d'un métal tendre dans un revêtement dur lors d'un procédé de dépôt physique en phase vapeur, ledit procédé comprenant les étapes suivantes :
fourniture d'un substrat ;
dépôt d'une couche d'adhérence sur le substrat ; et
dépôt du revêtement dur sur la couche d'adhérence par dépôt en phase vapeur, le métal tendre étant l'argent (Ag), déposé pour initier la formation de la couche, ladite formation étant interrompue afin d'éviter une coalescence complète, de manière à ce que le métal tendre forme des îlots contenus dans le revêtement dur constitué de nitrures, de carbures métalliques ou d'une combinaison de ceux-ci avec les métaux Ti, Zr, Ta, Al, Si, Cr.

2. Procédé selon la revendication 1, dans lequel le revêtement dur est TiN.

3. Procédé selon la revendication 2, dans lequel le revêtement comprend un revêtement AgTiN et une couche supérieure comprenant du TiN.

4. Procédé selon la revendication 3, dans lequel l'argent est déposé sous forme d'îlots dans le revêtement AgTiN.
